# EUROPEAN PATENT SPECIFICATION

(11) **EP 0 746 253 B1**
(45) Date of publication and mention of the grant of the patent: **02.05.2002**
(21) Application number: 94908616.9
(22) Date of filing: 19.01.1994
(51) Int. Cl.: A61B 17/58, A61F 2/28, F16B 35/04

(54) **TAPERED BONE SCREW WITH CONTINUOUSLY VARYING PITCH**
KONISCHE KNOCHENSCHRAUBE MIT VARIIERENDER GEWINDESTEIGUNG
VIS DE FIXATION OSSEUSE A PAS VARIABLE EN CONTINU

(30) Priority: 21.01.1993 US 7196
(43) Date of publication of application: 11.12.1996
(73) Proprietor: Acumed Inc., Beaverton, Oregon 97005 (US)
(72) Inventor: HUEBNER, Randall J., Acumed,Inc., Beaverton, OR 97005 (US); JENSEN, David, Glenn, Beaverton, OR 97005 (US); CONRAD, Gene, Lyle, Beaverton, OR 97005 (US)
(74) Representative: Allen, William Guy Fairfax
(86) International application number: PCT/US94/00738
(87) International publication number: WO 94/16636

(56) References cited:
- WO-A-89/09030
- WO-A-93/00518
- CH-A- 77 837
- DE-A- 3 630 863
- GB-A- 598 834
- US-A- 65 651
- US-A- 146 023
- US-A- 4 175 555
- US-A- 5 120 171

## Description

### 1. Field of the Invention

The present invention relates generally to a bone screw for drawing together portions of a bone and more particularly to such a screw which draws the bone portions together as a result of different-pitched threads on the screw.

### 2. Description of the Related Art

In healing bone fractures it is desirable to compress the fractures so that the fractured surfaces are pressed against one another. In the prior, art bone screws have been used to draw the fractured surfaces together and thereby optimize the healing process.

A number of prior art bone screws are constructed in similar to wood screws. For example, some prior art bone screws include a threaded distal portion and a head having a relatively long unthreaded shank disposed between the head and the distal portion. A drill is used to bore a hole through the fracture and the screw is threaded into the remote bone fragment with the head of the screw compressing the near fragment tightly against the remote bone fragment.

Other bone screws are threaded along the length thereof thus requiring a first drill bit to bore a hole in both bone fragments across the fracture and a second bit to drill a larger hole in the near bone fragment so that the screw threads do not engage the near bone fragment. Thereafter, the screw is tightened in the same manner as described above in connection with the screw having an unthreaded shank thereby compressing the fragments together.

Another bone screw is described in U.S. Patent No. 4,175,555 to Herbert. The Herbert bone screw includes a shaft having leading and trailing portions with a first screw thread formed on the leading portion. A second screw thread is formed on the trailing portion which is like-handed but of a smaller pitch than the first screw thread. A slot or hex socket is formed on the trailing portion to accommodate a driver for driving the screw into a bore formed across a bone fracture.

As noted in the Herbert patent, bone screws having heads suffer from several disadvantages including concentrated loads beneath the screw head and the protrusion of the screw head itself after the screw is installed. Several other shortcomings of the standard type of bone screw are detailed in the Herbert patent.

The Herbert bone screw is advantageous in that it eliminates a conventional screw head on the trailing end portion of the screw. The Herbert bone screw, however, itself suffers from a number of disadvantages.

In the Herbert screw, the leading threads have a smaller diameter than the trailing threads. This is necessary to permit the leading threads to pass through the relatively large bore in the near bone fragment and engage the smaller bore in the remote bone fragment. The larger trailing threads then engage the larger bore in the near bone fragment. As a result of this arrangement, any stripping of the threads cut into the bones during installation of the screw occurs in the remote bone. If the stripping occurred in the bore in the near bone fragment, a screw having a head thereon could still be used to compress the fracture even though the near bore was stripped; however, when stripping occurs in the bore in the remote bone, a standard screw with the head thereon cannot be used and another bore must be drilled.

Further, the Herbert screw must be correctly positioned, i.e., it is imperative that the fracture intersect the unthreaded central portion of the Herbert bone screw when the same is installed. In addition, because the Herbert screw is not threaded entirely along the length thereof, the purchase obtained by the screw in the bone is not as good as with a screw threaded along the entire length. Also, two bores of different sizes must be drilled to install the Herbert screw rather than a single bore.

Another instance where it is desirable to draw together portions of a bone for fusing the same together is in connection with arthrodesis of the interphalangeal joints. This procedure is sometimes indicated with symptoms of pain or instability in the finger joints. The purpose is to immobilize and draw together adjacent joints to cause them to fuse together thereby preventing further movement at the joint.

In one prior art procedure for immobilizing the distal interphalangeal joint (DIP), axial bores are drilled in the articular surfaces of the distal and proximal phalanges. The bore in the distal bone is sufficiently large to receive without threading a screw which is inserted thereinto via an incision in the tip of the finger. The screw threadably engages the bore in the proximal bone and when the screw head is tightened against the distal end of the distal bone, the two bones are compressed together. After several weeks, the bones fuse together. A second procedure to remove the screw must be performed because the head of the screw will cause discomfort in the finger pad if the screw is not removed.

This procedure is undesirable because it requires two separate surgeries. Katzman, et al, Use of Herbert Screw for Interphalangeal Joint Arthrodesis, clinical Orthopaedics and Related Research, No. 296 pages 127-132 (Nov. 1993), describes use of the screw disclosed in the Herbert patent in procedures for interphalangeal joint arthrodesis.

Many of the above-discussed disadvantages associated with using a Herbert screw to compress a fracture are also present when the Herbert screw is used for interphalangeal joint arthrodesis.

It would be desirable to provide a headless bone screw which overcomes the disadvantages associated with the Herbert bone screw.

A bone screw is also described in WO-A-9300518. In one embodiment the screw is cylindrical, except for a conical rear end. Over the conical portion, the pitch diminishes continuously toward the rear end.

According to the present invention, there is provided a bone screw comprising:
root portion with a leading end, a trailing end and a longitudinal axis extending therebetween;
a screw thread formed on the root portion, the screw thread extending from the leading end to the trailing end;
a crest formed on said screw thread, the crest having a radius measured from the longitudinal axis of the root portion; and
means on the trailing end of said root portion to accommodate a tool for driving the screw; wherein
the crest radius is larger near the trailing end and smaller near the leading end;
the maximum radius of the crest is larger than the maximum radius of any part of the screw; and
the screw thread has a pitch measured between corresponding points on consecutive thread crests, in which the pitch is larger near the leading end and smaller near the trailing end; characterised in that
the thread depth, measured as the radial distance between the root portion and the thread crest, near the leading end is at least substantially as large as the radius of the root portion and is larger than the thread depth near the trailing end.

The foregoing and other objects, features and advantages of the invention will become more readily apparent from the following detailed description of a preferred embodiment which proceeds with reference to the drawings, in which:-
Fig. 1 is an enlarged side elevation view of a bone screw constructed in accordance with the present invention shown partially in cross section.
Fig. 2 is an end view of the bone screw of Fig. 1.
Fig. 3 is a drawing illustrating the outside diameter of the screw.
Fig. 4 is a drawing illustrating the diameter of the root portion of the screw.
Fig. 5 is a cross-sectional view of a bone screw constructed in accordance with the present invention installed in a bone to draw a fracture together.
Fig. 6 is an enlarged side elevation view of a bone screw constructed in accordance with the present invention which may be used for interphalangeal joint arthrodesis.
Fig. 7 is a view of the bone screw of Fig. 6 installed in a distal interphalangeal joint with the bones forming the joint as shown in cross-section.

### DETAILED DESCRIPTION OF THE PREFERRED EMBODIMENT

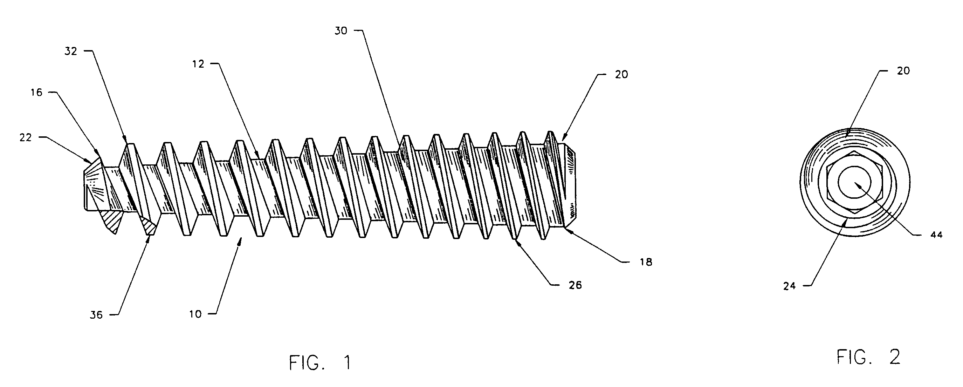

Indicated generally at 10 in Fig. 1 is a bone screw constructed in accordance with the present invention. Bone screw 10 is centered on a longitudinal axis 11. The length of screw 10 as measured along axis 11 is 1 cm (.394 inches) in the present embodiment of the invention. The bone screw includes a root portion 12 having a continuous screw thread 14 formed thereon.

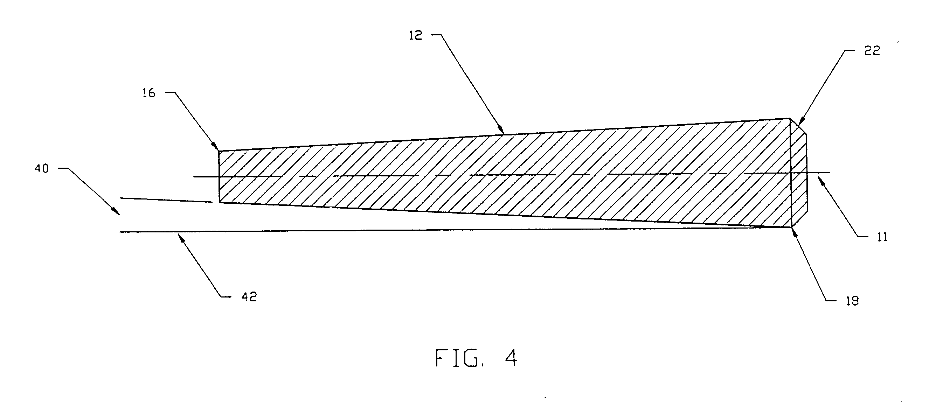

Root portion 12 includes a leading end 16 and a trailing end 18. As can best be seen in Fig. 4, the diameter of leading end 16 is less than the diameter of trailing end 18. Also in Fig. 4, it can be seen that root portion 12 tapers between trailing end 18 and leading end 16. A 45° bevel 20, in Fig. 1, is formed on trailing end 18. In the present embodiment of the invention, trailing end 18 has a diameter of approximately 0.234 cm (.092 inches). A frusto-conical nose portion 22 is formed on leading end 16 of root portion 12.

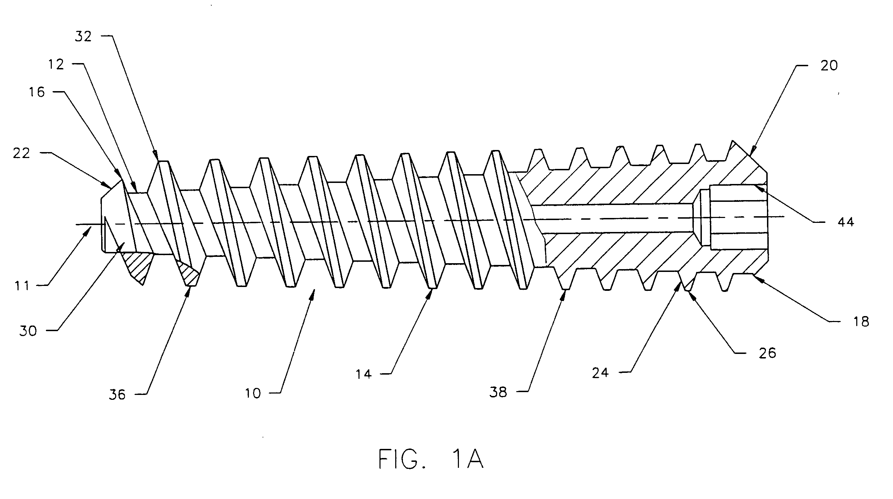

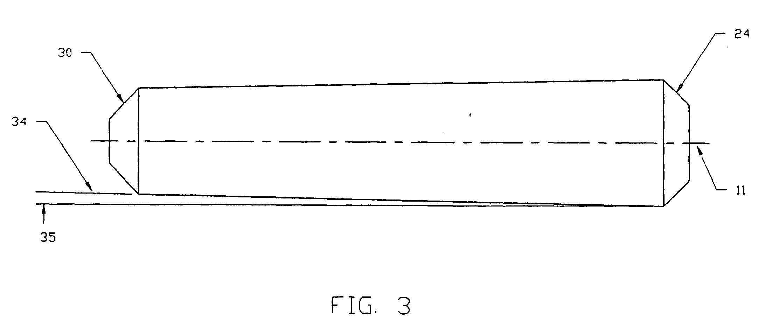

Screw thread 14 extends continuously between nose portion 22 and bevel 20. As can be seen in Figs. 2 and 3, a trailing thread 24 has a crest height, i.e., the distance between axis 11 and a crest 26 of trailing thread 24, which varies so as to form a substantially 45° angle, illustrated in Fig. 3, between the outside diameter of crest 24 and axis 11.

A similarly tapering leading thread 30 also has a crest 32 which varies in height over a first partial turn of screw thread 14 so as to form an angle of substantially 45° with axis 11 as illustrated in Fig. 3.

The crest of screw thread 14 between trailing and leading threads 24, 30 respectively, varies in height along the length of thread 14. In the present embodiment of the invention, the outside diameter defined by the crest of thread 14 between the leading and trailing threads forms an angle 34, in Fig. 3, of approximately 1.43" with respect to an axis 35 extending from the radially outermost portion of thread 14 parallel to axis 11. In the present embodiment of the invention, the diameter of the radially outermost portion of thread 14 is approximately 0.351 cm (.138 inches).

The pitch of thread 14, i.e., the distance from one point on the thread to the corresponding point on an adjacent thread measured parallel to axis 11, decreases between the leading and trailing ends of the screw. For example, in the present embodiment of the invention, the distance between the uppermost portion of crest 32 in Fig. 1 and a corresponding crest portion 36 is 0.12 609 cm (.04964 inches). The distance between the uppermost portion of crest 26 and a corresponding crest portion 38 is 0.12060 cm (.04748 inches). In the present embodiment of the invention, the pitch change per revolution is approximately 0.00091 cm (.00036 inches).

The pitch depth, i.e., the distance between the crest and the radially outer surface of root portion 12 similarly varies along the length of the screw. In the present embodiment of the invention, the pitch depth where leading thread 30 joins the remainder of screw thread 14 is approximately 0.0767 cm (.0302 inches). The pitch depth where trailing thread 24 joins the remainder of thread 14 is approximately 0.0610 cm (0.0240 inches).

The decrease in pitch depth between the leading end and trailing end of the screw can be seen by comparing Fig. 3 and Fig. 4 wherein root portion 12 tapers more sharply from the trailing to the leading end of the screw than does the change in crest height as shown in Fig. 3. In the present embodiment of the invention, the outside diameter of root portion 12 between leading and trailing ends, 16, 18, respectively, forms an angle 40, in Fig. 4, of approximately 2.5° with respect to an axis 42 extending from the radially outermost portion of trailing end 18 parallel to axis 11.

A hex socket 44 is formed on the trailing end of screw 10 to accommodate a driver as will be hereinafter further explained in connection with a description of the procedure in which the screw is used to draw opposing fragments of a fractured bone together.

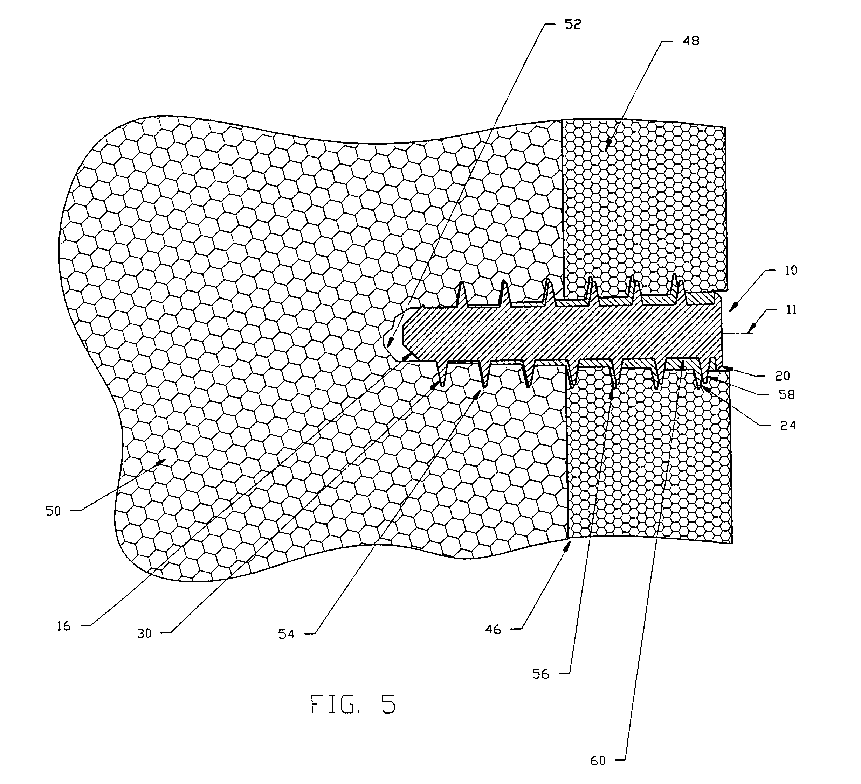

Turning now to Fig. 5, illustrated therein is a fracture 46 which separates adjacent bone portions 48, 50. Screw 10 is illustrated installed in a bore 52 which extends through bone portions 48, 50 across fracture 46.

In installing screw 10, a surgeon first drills bore 52 across bone portions 48, 50 as shown. The bit may be a conventional cylindrical bone bit or may comprise a bit having a slight taper from the leading to the trailing end thereof. Thereafter, the surgeon inserts a tool (not shown) having a hex driver extending therefrom which is connectable to hex socket 44 for screwing screw 10 into bore 52. Bore 52 is of a size to just receive leading end 16 of screw 10. As soon as nose portion 22 is received within the bore, torque is applied using the tool inserted into hex socket 44 thereby causing leading thread 30 to cut into the bone adjacent bore 52.

In the view of Fig. 5, screw 10 is hatched to show the path cut by leading thread 30 after screw 10 is installed in the position illustrated in Fig. 5. The path of thread 30 is depicted using hatching, like hatching 54, 56, 58 which indicated the position of the path cut by leading thread 30 relative to succeeding threads of the screw. Hatching 60 depicts the actual position of the thread on screw 10 and root 12. It is to be appreciated that hatching 54, 60 are not used in Fig. 5 to depict different structure, which is unitary as illustrated in Fig. 1, but to depict relative positions of the path cut by leading thread 30 in the actual position of subsequent threads in the installed screw.

Because of the decreasing pitch along the length of the screw, each successive thread received in the path cut by thread 30 exerts pressure against the right side (as viewed in Fig. 5) of the path cut by thread 30 thereby tending to compress the bone along the length of the screw As can be seen in Fig. 5, by the time the screw is fully installed, trailing thread 24 compresses a substantial amount of bone when it is received in the path cut by thread 30. This tends to draw bone portions 48, 50 tightly together across fracture 46 thereby promoting healing of the fracture.

As can be appreciated from the view of Fig. 5, the taper is important for two reasons. First, each succeeding portion of the thread is spaced further radially outwardly as a result of the taper and therefore the outer or portion of each thread (that portion closely adjacent the crest) cuts into new bone which was not cut by the preceding thread. This provides a much better purchase than would a thread having a continuously varying pitch on a cylindrical root. In such a configuration, each succeeding thread cuts additional bone within the generally cylindrical volume defined by the outside diameter of the threads. The outer portion of each thread (that portion closely adjacent the crest) therefore cuts into bone also cut by the preceding thread.

The tapered root is also advantageous in that the radially outer surface of the root, i.e., that portion between adjacent threads, is tightly urged against uncut bone defining the wall of bore 52. It is desirable for all surfaces of screw 10 to be tightly urged against adjacent bone, rather than a space cut by a thread, in order to increase purchase of the screw.

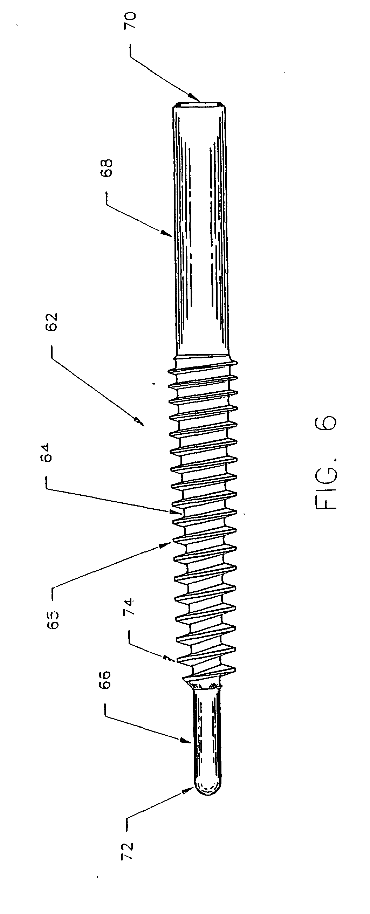

Turning now to Fig. 6, indicated generally at 62 is a second embodiment of a bone screw constructed in accordance with the present invention. Bone screw 62 is sized and constructed for use in connection with interphalangeal joint arthrodesis. Screw 62 includes a tapered root 64 having a thread 65 formed thereon, a substantially cylindrical leading end 66 and a substantially cylindrical trailing end 68. The diameter of leading end 66 is slightly larger than the leading end root 64 while the diameter of trailing end 68 is slightly smaller than the trailing end of the root. The trailing end 68 includes a hex socket (not visible), like hex socket 44 in Fig. 1A, formed on an end surface 70 thereof. Leading end 66 includes a tapered nose 72 formed on the forward end thereof. In the present embodiment of the invention, screw 62 is 3.101 cm (1.221 inches) in length with the threaded portion being 1.600 cm (.630 inches) long and the diameter of leading end 66 being 0.203cm (.080 inches). The trailing end diameter is 0.254 cm (.100 inches). As is the case with the previously described embodiment, the pitch of thread 65 decreases between the leading and trailing ends of the thread. In the embodiment of Fig. 6, a land 74 is formed in the crest of thread 65 and decreases in width between the leading and trailing edges of the screw.

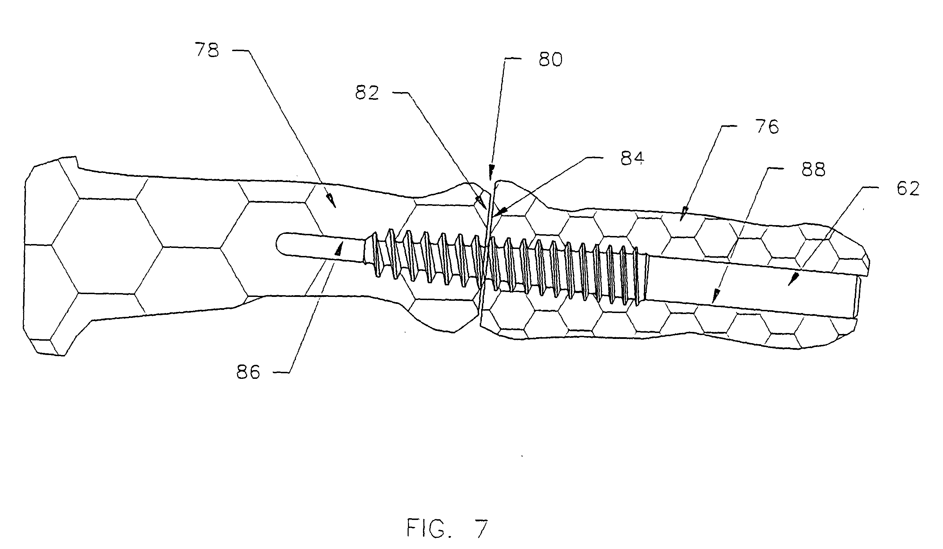

Turning now to Fig. 7, a distal phalanx 76 comprises the outermost bone of one of the four fingers. A proximal phalanx 78 is adjacent thereto with a distal interphalangeal (DIP) joint 80 being formed therebetween.

The joint includes a pair of articular surfaces 82, 84 which have been flattened in accordance with a known technique for immobilizing DIP 80. Bores 86, 88 are drilled into each of phalanxes 76, 78 from articular surfaces 82, 84, respectively. Thereafter the bones are repositioned as shown in Fig. 7 and screw 62 is driven into the distal end of the bore in phalanx 76 until the screw is positioned as shown in Fig. 7.

Screw 62 thus compresses across 80 even though it has a relatively small diameter, which is critical in DIP joint arthrodesis, and also has sufficient length, due to the leading and trailing ends 66, 68, to provide stability while the bones are fusing. Because the screw is entirely received within the bones; i.e., there is no protrusion from the screw, it can remain implanted and thus a second procedure to remove the screw is not necessary.

## Claims

1. A bone screw comprising:
a root portion (12) with a leading end (16), a trailing end (18) and a longitudinal axis (11) extending therebetween;
a screw thread (14) formed on the root portion (12), the screw thread extending from the leading end to the trailing end;
a crest (26, 36) formed on said screw thread, the crest having a radius measured from the longitudinal axis of the root portion; and
means (44) on the trailing end of said root portion to accommodate a tool for driving the screw; wherein
the crest radius is larger near the trailing end (18) and smaller near the leading end (16);
the maximum radius of the crest (26, 36) is larger than the maximum radius of any part of the screw; and
the screw thread (14) has a pitch, measured between corresponding points on consecutive thread crests, in which the pitch is larger near the leading end (16) and smaller near the trailing end (18); **characterised in that**
the thread depth, measured as the radial distance between the root portion (12) and the thread crest, near the leading end (16) is at least substantially as large as the radius of the root portion (12) and is larger than the thread depth near the trailing end (18).

2. A screw according to claim 1, **characterised in that** the pitch gradually increases as the crest radius gradually decreases.

3. A screw according to claim 1 or 2, **characterised in that** the crest radius gradually tapers over the root portion (12).

4. A screw according to claim 1, 2 or 3, **characterised in that** the root portion (12) has a radius which is smaller near the leading end (16) and larger near the trailing end (18).

5. A screw according to any preceding claim, **characterised in that** the radial distance between the root portion and the crest (26, 36) is larger near the leading end (16), where the crest radius is smaller, than it is near the trailing end (18), where the crest radius is larger.

6. A screw according to any preceding claim wherein the trailing end (18) is a drive end, and said means on the trailing end comprises a drive socket (44), with a hole extending axially forward from the drive socket (44) toward the leading end (16) of the root portion (12).

7. A screw according to any preceding claim, **characterised in that** an unthreaded extension (66; 68) extends from one of the ends of the root portion (64).

8. A screw according to any preceding claim, **characterised in that** the crest radius tapers at a substantially constant rate.

9. A screw according to any preceding claim, **characterised in that** the root portion (12) has a substantially constant taper between the leading (16) and trailing ends (18).

10. A screw according to any preceding claim, **characterised in that** the pitch varies substantially uniformly between the leading (16) and trailing ends (18).

11. A screw according to any preceding claim, **characterised in that** the thread depth increases substantially uniformly from the trailing end (18) to the leading end (16).

## Patentansprüche

1. Knochenschraube, enthaltend:
einen Wurzelbereich (12) mit einem vorlaufenden Ende (16), einem nachlaufenden Ende (18) und einer sich dazwischen erstreckenden Längsachse (11);
ein an dem Wurzelbereich (12) ausgebildetes Schraubgewinde (14), das sich von dem vorlaufenden Ende zu dem nachlaufenden Ende erstreckt;
einen an dem Schraubgewinde ausgebildeten Scheitel (26, 36), der gemessen von der Längsachse des Wurzelbereiches aus einen Radius hat; und
eine Einrichtung (44) an dem nachlaufenden Ende des Wurzelbereiches zur Aufnahme eines Werkzeugs zum Antreiben der Schraube; wobei
der Scheitelradius nahe dem nachlaufenden Ende (18) größer ist und nahe dem vorlaufenden Ende (16) kleiner ist;
der maximale Radius des Scheitels (26, 36) größer ist als der maximale Radius irgendeines Teils der Schraube; und
das Schraubgewinde (14) eine zwischen entsprechenden Stellen an aufeinanderfolgenden Gewindescheiteln gemessene Steigung hat, wobei die Steigung nahe dem vorlaufenden Ende (16) größer und nahe dem nachlaufenden Ende (18) kleiner ist,
**dadurch gekennzeichnet, daß**
die Gewindetiefe, gemessen als radialer Abstand zwischen dem Wurzelbereich (12) und dem Gewindescheitel, nahe dem vorlaufenden Ende (16) zumindest im wesentlichen so groß ist wie der Radius des Wurzelbereiches (12) und größer ist als die Gewindetiefe nahe dem nachlaufenden Ende (18).

2. Schraube nach Anspruch 1, **dadurch gekennzeichnet, daß** die Steigung allmählich zunimmt entsprechend wie der Scheitelradius allmählich abnimmt.

3. Schraube nach Anspruch 1 oder 2, **dadurch gekennzeichnet, daß** der Scheitelradius sich allmählich über den Wurzelbereich (12) verjüngt.

4. Schraube nach Anspruch 1, 2 oder 3, **dadurch gekennzeichnet, daß** der Wurzelbereich (12) einen Radius hat, der nahe dem vorlaufenden Ende (16) kleiner ist und nahe dem nachlaufenden Ende (18) größer ist.

5. Schraube nach einem der vorhergehenden Ansprüche, **dadurch gekennzeichnet, daß** der radiale Abstand zwischen dem Wurzelbereich und dem Scheitel (26, 36) größer nahe dem vorlaufenden Ende (16) ist, wo der Scheitelradius kleiner ist, als er nahe dem nachlaufenden Ende (18) ist, wo der Scheitelradius größer ist.

6. Schraube nach einem der vorhergehenden Ansprüche, wobei das nachlaufende Ende (18) ein Antriebsende ist und die Einrichtung an dem nachlaufenden Ende eine Antriebsbuchse (44) enthält, wobei sich von der Antriebsbuchse (44) in Richtung zu dem vorlaufenden Ende (16) des Wurzelbereiches (12) ein Loch axial nach vorne erstreckt.

7. Schraube nach einem der vorhergehenden Ansprüche, **dadurch gekennzeichnet, daß** von einem der Enden des Wurzelbereiches (64) eine gewindelose Verlängerung (66; 68) ausgeht.

8. Schraube nach einem der vorhergehenden Ansprüche, **dadurch gekennzeichnet, daß** der Scheitelradius sich in einem im wesentlichen konstanten Maß verjüngt.

9. Schraube nach einem der vorhergehenden Ansprüche, **dadurch gekennzeichnet, daß** der Wurzelbereich (12) zwischen dem vorlaufenden Ende (16) und dem nachlaufenden Ende (18) eine im wesentlichen konstante Konizität aufweist.

10. Schraube nach einem der vorhergehenden Ansprüche, **dadurch gekennzeichnet, daß** die Steigung sich zwischen dem vorlaufenden Ende (16) und dem nachlaufenden Ende (18) im wesentlichen gleichmäßig verändert.

11. Schraube nach einem der vorhergehenden Ansprüche, **dadurch gekennzeichnet, daß** die Gewindetiefe von dem nachlaufenden Ende (18) zu dem vorlaufenden Ende (16) im wesentlichen gleichmäßig zunimmt.

## Revendications

1. Vis de fixation osseuse comprenant
une partie formant fond de filet (12) avec une extrémité avant (16), une extrémité arrière (18) et un axe longitudinal (11) s'étendant entre ces deux extrémités,
un filet de vis (14) réalisé sur la partie formant fond de filet (12), le filet de vis s'étendant de l'extrémité avant jusqu'à l'extrémité arrière,
un sommet (26, 36) réalisé sur ledit filet de vis et ayant un rayon qui est mesuré à partir de l'axe longitudinal de la partie formant fond de filet, et
un moyen (44) prévu sur l'extrémité arrière de ladite partie formant fond de filet aux fins de recevoir un outil pour entraîner la vis, sachant que
le rayon du sommet est plus grand à proximité de l'extrémité arrière (18) et plus petit à proximité de l'extrémité avant (16),
le rayon maximal du sommet (26, 36) est plus grand que le rayon maximal de toutes les autres parties de la vis, et
le filet de vis (14) présente un pas mesuré entre des points correspondants sur des sommets de filet consécutifs, le pas étant plus grand à proximité de l'extrémité avant (16) et plus petit à proximité de l'extrémité arrière (18), **caractérisée par le fait que**
la profondeur de filet, mesurée en tant que distance radiale entre la partie formant fond de filet (12) et le sommet de filet, à proximité de l'extrémité avant (16), est au moins sensiblement aussi grande que le rayon de la partie formant fond de filet (12) et est plus grande que la profondeur de filet à proximité de l'extrémité arrière (18).

2. Vis selon la revendication 1, **caractérisée par le fait que** le pas augmente progressivement à mesure que le rayon du sommet diminue progressivement.

3. Vis selon la revendication 1 ou 2, **caractérisée par le fait que** le rayon du sommet diminue progressivement le long de la partie formant fond de filet (12).

4. Vis selon la revendication 1, 2 ou 3, **caractérisée par le fait que** la partie formant fond de filet (12) présente un rayon qui est plus petit à proximité de l'extrémité avant (16) et plus grand à proximité de l'extrémité arrière (18).

5. Vis selon l'une des revendications précédentes, **caractérisée par le fait que** la distance radiale entre la partie formant fond de filet et le sommet (26, 36) est plus grande à proximité de l'extrémité avant (16), où le rayon du sommet est plus petit, qu'à proximité de l'extrémité arrière (18) où le rayon du sommet est plus grand.

6. Vis selon l'une des revendications précédentes, dans laquelle l'extrémité arrière (18) est une extrémité d'entraînement et ledit moyen prévu sur l'extrémité arrière comporte une cavité d'entraînement (44), un trou s'étendant axialement de la cavité d'entraînement (44) en direction de l'extrémité avant (16) de la partie formant fond de filet (12).

7. Vis selon l'une des revendications précédentes, **caractérisée par le fait qu'**un prolongement (66, 68) non fileté s'étend depuis l'une des extrémités de la partie formant fond de filet (64).

8. Vis selon l'une des revendications précédentes, **caractérisée par le fait que** le rayon du sommet diminue de manière sensiblement constante.

9. Vis selon l'une des revendications précédentes, **caractérisée par le fait que** la partie formant fond de filet (12) diminue de section de façon sensiblement constante entre l'extrémité avant (16) et l'extrémité arrière (18).

10. Vis selon l'une des revendications précédentes, **caractérisée par le fait que** le pas varie de manière sensiblement uniforme entre l'extrémité avant (16) et l'extrémité arrière (18).

11. Vis selon l'une des revendications précédentes, **caractérisée par le fait que** la profondeur du filet augmente de manière sensiblement uniforme depuis l'extrémité arrière (18) jusqu'à l'extrémité avant (16).
